# EUROPÄISCHE PATENTSCHRIFT

(11) **EP 1 779 872 B1**
(45) Veröffentlichungstag und Bekanntmachung des Hinweises auf die Patenterteilung: **29.02.2012**
(21) Anmeldenummer: 06021929.2
(22) Anmeldetag: 19.10.2006
(51) Int. Cl.: A61L 9/12, A01M 1/20

(54) **Abgabesystem für die kontinuierliche Freisetzung flüchtiger Substanzen**
Diffuser for the continuous release of volatile substances
Diffuseur pour l'évaporation continue de substances volatiles

(30) Priorität: 29.10.2005 DE 102005051892
(43) Veröffentlichungstag der Anmeldung: 02.05.2007
(73) Patentinhaber: LTS LOHMANN Therapie-Systeme AG, 56626 Andernach (DE)
(72) Erfinder: Kocherscheidt, Birgit, 56579 Bonefeld (DE); Jäckels, Hermann-Josef, 56170 Bendorf (DE); Beitzel, Else, 56626 Andernach (DE); Roreger, Michael, 56567 Neuwied (DE); Kloczko, Malgorzata, 53577 Neustadt/Wied (DE)
(74) Vertreter: Siemund, Volker

(56) Entgegenhaltungen:
- WO-A-00/08095
- US-A- 1 394 497
- US-A- 3 027 678

## Beschreibung

Gegenstand der Erfindung ist ein Abgabesystem für die kontinuierliche Freisetzung von flüchtigen Substanzen, insbesondere Duftstoffen, Repellentien, Lockstoffen und / oder Pheromonen, bei dem eine die freizusetzende(n) Substanz(en) enthaltende Polymermatrix in aufgewickelter Form vorliegt.

Zum Stand der Technik zählen Abgabesysteme wie die RAK-Dispenser (Vertrieb durch die Fa. Leu + Gygax AG, 5413 Birmenstorf, Schweiz) oder die Produkte aus der Isomate-Reihe (Vertrieb durch die Fa. Andermatt Biocontrol AG, 6146 Grossdietwil, Schweiz). Hierbei handelt es sich um mit flüssigen Lockstoffen befüllte Kunststoffampullen bzw. um Drähte, die mit Lockstoffe enthaltenden Polymeren ummantelt sind.

In US 1,394,497 wird ein Insektenvernichter offenbart, der aus gewellter Pappe besteht, die mit Arsen als Kontaktgift sowie Zucker und Honig als Lockstoff imprägniert ist. Die Wellpappe ist aufgerollt und befindet sich in einem verschlossenen zylindrischen Behälter, der an der Stirnseite Öffnungen besitzt. Ameisen und andere Insekten können durch diese Öffnungen in den Behälter eindringen und sich in den Kammern der Wellpappe bewegen. Es ist dazu erforderlich, dass die zu vernichtenden Insekten in den Behälter eindringen, um mit dem Gift in Kontakt zu kommen und vernichtet zu werden.

US 3,027,678 bezieht sich auf eine Vorrichtung zur Verbreitung von Pestiziden und anderen flüchtigen Substanzen an die Luft. Der Gegenstand umfasst ein zylindrisches Gehäuse und eine mit Pestizid beladene, kleine Öffnungen aufweisende Struktur. Als Material für diese Struktur kommen Glaswolle, Baumwolle und auch gerolltes Filterpapier in Frage. Eine abziehbare Membran am Ende des Gehäuses ermöglicht die Freisetzung aus der Struktur.

Nach WO 00/08095 werden duftende Polymere dadurch hergestellt, dass ein zerkleinertes erstes Polymermaterial mit einem Duftstoff gemischt, aufquellen gelassen und anschließend unter kontrollierten Bedingungen mit einem zweiten Polymermaterial zur weiteren Verarbeitung gemischt wird.

Diese Systeme haben oft den Nachteil, dass die flüchtige Substanz in einem verhältnismäßig kurzen Zeitraum freigesetzt wird und das Abgabesystem daher recht bald nach Beginn seiner Anwendung durch ein neues ersetzt werden muss.

Aufgabe der Erfindung ist es, ein Abgabesystem für die kontinuierliche Freisetzung von Substanzen zur Verfügung zu stellen, das befähigt ist, die betreffende Substanz über einen längeren Zeitraum freizusetzen und das einfach herzustellen ist.

Gelöst wird die Aufgabe durch ein Abgabesystem gemäß Anspruch 1 das eine Polymermatrix umfasst, die die freizusetzende Substanz enthält und die in dem Abgabesystem in aufgewickelter Form vorliegt. Aus dem Abgabesystems kann die freizusetzende Substanz kontinuierlich und über einen längeren Zeitraum freigesetzt werden.

In einer weiteren Ausführungsform kann das Abgabesystem auch einen Träger für die Polymermatrix enthalten, der vorzugsweise bahnförmig ist und als solcher ebenfalls in aufgewickelter Form vorliegen kann.

Weitere Bestandteile des Abgabesystems können ein Kern und / oder eine Befestigungsvorrichtung sein.

Als Kern kann ein drehbarer Körper dienen, der vorzugsweise zylindrisch ist und um den herum die Polymermatrix aufgewickelt wird. Als Material eines solchen Körpers können beispielsweise Metalle, Kunststoffe und Naturstoffe dienen. Diese Körper liegen vorzugsweise in Form eines Stäbchens oder eines Drahts vor. Der drehbare Körper kann in einer besonderen Ausführungsform auf zumindest einer Seite über die Breite der Polymermatrix hinausgehen und an seinem Ende hakenförmig gebogen sein. In dieser Ausführungsform kann der drehbare Körper nicht nur den Kern der Wicklung bilden, sondern auch zugleich als Befestigungsvorrichtung dienen.

Als Befestigungsvorrichtung können jedoch auch neben dieser Ausführungsform eines hakenförmig endenden Kerns auch andere Vorrichtungen dienen, beispielsweise Schellen, die um die Wicklung herum verlaufen oder Körbe, in die das Abgabesystem eingesetzt wird, welche ihrerseits wieder vorzugsweise mit Haken versehen sind. Die Befestigungsvorrichtung ist dabei so ausgerüstet, dass sie vorzugsweise mit einem Handgriff sicher, d. h. schlagfest, am Zielobjekt (beispielsweise eine Weinrebe oder der Zweig eines Obstbaums) befestigt werden kann.

Das Abgabesystem besitzt eine Höhe, die mindestens der Breite des Trägers, ggf. zuzüglich des überstehenden Teils einer eventuell vorhandenen Befestigungsvorrichtung entspricht. Bevorzugt ist das Abgabesystem bis zu 20 cm hoch, vorzugsweise 3 bis 10 cm. Der Durchmesser des Abgabesystems ist im Wesentlichen von der Dicke der Polymermatrix und der Zahl der Wicklungen abhängig. Vorzugweise liegt er zwischen 0,5 und 5 cm, besonders bevorzugt zwischen 1 und 2 cm.

Als Verpackung für das Abgabesystem kommen vorzugsweise Behältnisse in Frage, die undurchlässig für die freizusetzende Substanz sind. Hierzu zählen beispielsweise Schlauchbeutel, die aus einem für die freizusetzende Substanz undurchlässigem Material wie Surlyn gefertigt sind.

Die Polymermatrix ist aus Materialien aufgebaut, die befähigt sind, die freizusetzende Substanz aufzunehmen und sie wieder freizusetzen. Geeignete Materialien sind synthetische Polymere.

Zu den natürlichen Polymeren zählen Polysaccharide wie Stärke, Cellulose, Chitin, Pektine, Cellulose, Hemicellulosen etc., Polypeptide wie Keratine, Gelatine, Kollagen, Proteine etc. und Polyprene wie Naturkautschuk.

Zu den synthetischen Polymeren zählen chemisch modifizierte Derivate der genannten natürlichen Polymere, d. h. abgewandelte Naturstoffe.

Weiterhin zählen zu den synthetischen Polymeren solche, die durch Polykondensation, Polymerisation oder Polyaddition geeigneter Monomere herstellbar sind. Zu den Polykondensaten zählen Polyamide, Polycarbonate, Polyester und Polyvinylacetale. Zu den Polymerisaten zählen Polyethylene, Polypropylene, Poly-1-butene, Poly-4-methyl-1-pentene, Polyvinylchloride, Poly(meth)acrylate, Polyacrylnitrile, Polystyrole, Polyacetale, Fluor-Kunststoffe, Polyvinylalkohole und Polyvinylacetate. Zu den Polyaddukten zählen Polyurethane.

Innerhalb der Gruppe der synthetischen Polymeren sind die thermoplastischen Kunststoffe eine besonders bevorzugte Gruppe von geeigneten Materialien für die Polymermatrix.

Besonders bevorzugt werden Kautschuk, Dreiblock-Polymere (z. B. Polystyrol-Blockcopolymere wie z. B. Kraton G), Polyurethane, Ethylen-Vinylacetat-Copolymere (z. B. Evatane 40-55), Polyacrylate (z. B. solche auf Lösemittelbasis wie Durotak 380-2954, oder als Festkörper im Handel erhältliche wie Durotak H 312), Polyamide, Polyisobutene (z. B. solche mit einer Molmasse zwischen 40.000 und 120.000 g/mol und solche mit einer Molmasse zwischen 300.000 und 2.500.000 g/mol sowie deren Gemische), Polyvinylalkohole und Polyvinylacetate als Material für die Polymermatrix verwendet.

Die Polymermatrix kann neben dem bzw. den synthetischen Polymer(en) auch Weichharze, Hartharze und / oder Hilfsstoffe enthalten. Als Hilfsstoffe können Cellulosederivate, Sunblocker, Reflektoren, Verdickungsmittel, Füllstoffe, Weichmacher, Klebrigmacher (tackifier), Kohäsionsverbesserer, Farbstoffe, Pigmente etc. dienen.

Der Gesamtgehalt der Hilfsstoffe kann bis zu 50 Gew.-%, vorzugsweise bis zu 30 Gew.-% der Polymermatrix betragen.

Die Weichharze dienen im Wesentlichen zur Einstellung einer gewissen Klebrigkeit, welche zur Gewährleistung der strukturellen Integrität der Polymermatrix und somit zur Verhinderung des Entrollens der Wicklung beitragen kann. Unter dem Begriff Weichharz versteht man natürliche Harze oder synthetische Harze, die bei Normaltemperatur flüssig sind. Sie können zu einem festen Film trocknen oder als Film klebrig bleiben. Die nicht trocknenden Weichharze können auch den Weichmachern zugerechnet werden. Es handelt sich dabei chemisch gesehen um mittel- bis höhermolekulare Polyolefine, Polyester, Polyether, Polyacrylate oder Aminoplaste. Einige davon werden wegen ihrer Eigenschaften und Funktionen bei der Anwendung auch als Oligomer- oder Polymer-Weichmacher bzw. als Weichmacherharze bezeichnet.

In Abhängigkeit von dem Material, das für die Polymermatrix gewählt wird, kann der Gehalt an Weichharz zwischen 0 und 50 Gew.-% liegen, vorzugsweise zwischen 20 und 40 Gew.-%. Zu den bevorzugten Weichharzen zählen Produkte, wie beispielsweise Terpenphenolharze, die unter den Markennamen Dertophene erhältlich sind oder Hydroabietylalkoholharze, die unter dem Markennamen Abitol erhältlich sind.

Zu den Hartharzen rechnet man neben Kolophonium und seinen Derivaten auch höherschmelzende Umsetzungsprodukte von Harzsäuren, also Harzester sowie die Additionsprodukte von Kolophonium und Maleinsäureanhydrid (Maleinat-Harze), die als Kunstkopale bezeichneten harzmodifizierten Phenolharze und die Aldehyd- und Keton-Harze. Als bevorzugtes Hartharz kommt beispielsweise Herculin C in Frage. Der Gehalt an Hartharzen kann zwischen 0 und 20 Gew.-% liegen.

Die Polymermatrix kann in einer besonderen Ausführungsform klebend, insbesondere auch haftklebend ausgerüstet sein. Unter "klebend" wird hier die Eigenschaft verstanden, dass eine Haftwirkung zwischen der Oberfläche der Polymermatrix und einem Substrat auftritt, die durch mechanische Verbindung, Diffusion in der Übergangszone, Adsorption, elektrostatische Kräfte, van-der-Waals-Kräfte oder durch chemische Bindung bedingt ist. Unter "haftklebend" ist zu verstehen, dass die klebende Eigenschaft im lösungsmittelfreien Zustand bei 20 °C erhalten bleibt, so dass die Polymermatrix permanent klebend ist und klebfähig bleibt. Im "haftklebend" ausgerüsteten Zustand kann die Polymermatrix mit leichtem Anpressdruck auf fast allen Substraten haften und davon im Wesentlichen auch wieder rückstandsfrei abgezogen werden. Generell kann die Klebrigkeit der Polymermatrix durch die Wahl geeigneter Weich- und / oder Hartharze und deren Anteil in der Polymermatrix eingestellt werden.

Die Polymermatrix kann farblos oder farbig sein. Sie kann auch transparent oder lichtundurchlässig sein.

Die Polymermatrix kann bahnförmig und in unterschiedlichen Schichtdicken, vorzugsweise jedoch mit einer konstanten Schichtdicke vorliegen. Sie kann zwischen etwa 25 µm und 1 mm dick sein. Vorzugsweise weist die Polymermatrix eine Schichtdicke zwischen 100 und 500 µm auf.

Die Länge der Polymermatrix in einem konkreten Abgabesystem kann von verschiedenen Faktoren abhängen, z. B. dem Gehalt der freizusetzenden Substanz und der gewünschten Abgaberate, der Breite der Polymermatrix (a) und der Handhabbarkeit des Abgabesystems. Als technisch bedingte Untergrenze der Länge ist ein Wert von 1 cm anzusehen. Als technisch sinnvolle Obergrenze der Länge ist ein Wert von etwa 200 m anzusetzen. Bevorzugte Längenbereiche liegen zwischen 5 cm und 50 m, vorzugsweise zwischen 20 cm und 5 m.

Die Breite der Polymermatrix (a) kann oberhalb von 2 mm liegen. Die Obergrenze der Breite hängt von der Handhabbarkeit des Abgabesystems ab und sollte nicht oberhalb von 50 cm liegen. Bevorzugte Breiten liegen zwischen 5 mm und 40 cm, besonders bevorzugt zwischen 2 cm und 40 cm.

Als Träger kommen Materialien in Frage, die in flächiger Form vorliegen können. Geeignete Materialien sind Vliesstoffe (z. B. aus Polyethylen, Polyethylenterephthalat, Polypropylen, Viskose und / oder deren Gemischen), Metallfolien (z. B. aus Aluminium), Polyethylen (PE), orientiertes Polypropylen (OPP), Polypropylen (PP), Polyurethan (PUR), Papier, Schaumstoffe (z. B. offenzellige, geschlossenzellige etc. aus Gerüstsubstanzen wie z. B. Polystyrol, Polyurethan, Polyvinylchlorid etc.), Glasfasern und textile Werkstoffe sowie Kombinationen oder Verbundmaterialien davon.

Der Träger hat - sofern er vorhanden ist - die Funktion, die darauf befindliche Polymermatrix zu tragen und ihr damit einen zusätzlichen Halt zu verschaffen. Der Träger liegt daher vorzugsweise in Form einer Folie, ggf. auch in Form eines Verbundlaminats vor. Der Träger kann auch bei der Herstellung des Abgabesystems als zu beschichtende Substratbahn dienen, auf die eine Masse enthaltend zumindest das Polymermaterial der Polymermatrix und die freizusetzende Substanz aufgetragen wird.

Das Trägermaterial kann frei von der freizusetzenden Substanz sein. Er kann vorzugsweise aber auch für die freizusetzende Substanz permeabel sein. In dieser Ausführungsform kann der Träger auch die Diffusionsgeschwindigkeit der freizusetzenden Substanz auf dem Weg von "kernnahen Wicklungen" an die äußere Oberseite (d. h. die Außenseite der letzten Wicklung) des Abgabesystems kontrollieren. Mit diesen Eigenschaften wirkt der Träger als Membran.

Der Träger kann in einer besonderen Ausführungsform bedruckt sein.

In einer besonderen Ausführungsform bedeckt der Träger die Polymermatrix einseitig vollflächig, wobei Träger und Polymermatrix ein Verbundlaminat oder zumindest einen Bestandteil davon bilden. In diesem Fall sind Länge und Breite des Trägers identisch mit der jeweiligen Länge und der jeweiligen Breite der Polymermatrix. Der Träger kann jedoch auch eine Breite (b) ausweisen, die von der Breite der Polymermatrix (a) abweicht. Vorzugsweise ist die Breite des Trägers (b) größer als die der Polymermatrix, z. B. damit diese vor äußeren physikalischen oder mechanischen Einflüssen besser geschützt wird.

Der Träger kann jedoch auch eine wesentlich kürzere Länge aufweisen, z. B. falls er die Polymermatrix nicht vollflächig bedeckt. So kann der Träger in einer besonderen Ausführungsform die in aufgewickelter Form vorliegende Polymermatrix nur einmal umhüllen und auf diese Weise praktisch als "letzte Abschlusslage" eine Ummantelung der Polymermatrix darstellen.

Als Träger kommen vorzugsweise kommerziell erhältliche Kunststoffe (beispielsweise aus Polyethylen, Polypropylen, Polyurethan, Polystyrol etc.) und Cellulose- und deren Derivate sowie deren Kombinationen in Frage. Der Träger kann in der Form einer Folie, einer Vliesstoffschicht, eines Verbundwerkstofflaminats, einer festen Schaumstoffschicht etc. vorliegen. Eine bevorzugte Ausführungsform ist ein mit Polyethylen und / oder Polypropylen beschichtetes Papier.

Die Schichtdicke des Trägers kann zwischen 40 und 100 µm liegen, vorzugsweise zwischen 50 und 70 µm.

Als freizusetzende Substanzen, die für das Abgabesystem verwendet werden, kommen flüchtige Substanzen in Frage. Hierzu zählen insbesondere Lockstoffe, Repellentien und Duftstoffe sowie deren Kombinationen. Zu den Lockstoffen zählen insbesondere Sexuallockstoffe (Pheromone) und Fraßlockstoffe. Zu den Repellentien zählen Mittel zur Abschreckung von Insekten und Spinnen (Insektenabwehrmittel) und Fraßhemmstoffe. Zu den Insektenabwehrmitteln zählen etherische Öle wie Anisöl, Bergamottöl, Birkenholzteer, Campher, Citronellöl, Eucalyptusöl, Geraniumöl, Kiefernöle, Kokosnußöl, Lavendelöl, Muskatnußöl, Nelkenöl, Orangenblütenöl, Pfefferminzöl, Poleiöle (Pennyroyalöl), Pyrethrum, Thymianöl und Zimtöl. Auch synthetische Repellentien sind bekannt, wie beispielsweise N,N-Diethyl-m-toluamid, Dimethylcarbamat, 2-Ethyl-1,3-hexandiol, Octansäurediethylamid, 2-(Octylthio)ethanol und 3-(N-Acetyl-N-butylamino)propionsäureethylester. Als Duftstoffe kommen die dem Fachmann bekannten etherischen Öle und synthetischen Riechstoffe in Frage. Als freizusetzende Substanz kann auch die Kombination von mindestens zwei Lockstoffen, mindestens zwei Repellentien oder mindestens zwei Duftstoffen eingesetzt werden.

Der Gehalt der freizusetzenden Substanz in der Polymermatrix kann von der gewünschten Freisetzungsdauer, den geometrischen Eigenschaften des Abgabesystems, den physiko-chemischen Eigenschaften des Material, aus dem die Polymermatrix aufgebaut ist, der physiologischen Wirkung der freizusetzenden Substanz u. a. abhängen. Üblicherweise liegt der Gehalt der freizusetzenden Substanz zwischen 0,001 und 60 Gew.-%, vorzugsweise zwischen 0,1 und 35 Gew.-%.

Besonders bevorzugte freizusetzende Substanzen sind (Z)9-Dodecenylacetat, Dodecylacetat, Octadecylacetat, (E,Z)-7,9-Dodecadienylacetat, (E,E)-8,10-Dodecadien-1-ol, Dodecan-1-ol, Tetradecan-1-ol und deren Mischungen; insbesondere die, welche als spezifische Pheromone für Eupoecilia ambiguella bzw. Lobesia botrana verwendet werden können.

Unter dem Begriff "freizusetzende Substanz" im Sinne dieser Beschreibung ist selbstverständlich auch die Kombination von zwei oder mehr Substanzen zu verstehen, die die genannten Kriterien erfüllen. Die freizusetzende Substanz ist in der Polymermatrix zumindest teilweise gelöst enthalten. Ungelöste Bestandteile der freizusetzenden Substanz liegen vorzugsweise in suspendierter Form in der Polymermatrix vor.

Unter dem Begriff "in aufgewickelter Form" ist zu verstehen, dass die Polymermatrix in bahnförmigem Zustand einem als "Wickeln" bezeichneten Verfahrensschritt unterworfen wird und im Ergebnis dann als eine aufgewickelte Rolle vorliegt. Äußerlich betrachtet bildet diese Polymermatrix einen Zylinder mit einer definierten Höhe (die sich aus der Breite der eingesetzten bahnförmigen Polymermatrix ergibt) und einem definierten Durchmesser (der von der Schitdicke der Polymermatrix und der Zahl der Wicklungen abhängt). Die Zahl der Wicklungen der in aufgewickelter Form vorliegenden Polymermatrix ist nach oben hin theoretisch unbegrenzt; nach unten ist die minimale Zahl der Wicklungen bei 1, was einer doppellagige Polymermatrix entspricht. In der Praxis ist Obergrenze der Wicklungen nicht größer als 2000. Vorzugsweise liegt die Zahl der Wicklungen zwischen 10 und 500.

Unter dem Begriff "kontinuierliche Freisetzung" ist zu verstehen, dass die freizusetzende Substanz nach "Aktivierung" (d. h. nach dem Auspacken des Abgabesystems aus der Verpackung) ohne zeitliche Unterbrechung aus dem Abgabesystem an die Umgebung abgegeben wird. Die Freisetzung der freizusetzenden Substanz kann über einen längeren Zeitraum von mindestens 1 Tag andauern. Vorzugsweise dauert die Freisetzung jedoch mindestens eine Woche oder noch länger an. In einer bevorzugten Ausführungsform ist das Abgabesystem befähigt, die freizusetzende Substanz über einen Zeitraum von mindestens einem Monat abzugeben.

Einflussgrößen für die Abgaberate sind die Dicke der Polymermatrix, der Gehalt der freizusetzenden Substanz in der Polymermatrix (= Beladung), das eventuelle Vorhandensein eines als Membran wirkenden Trägers, die Permeabilität des Trägers für die freizusetzende Substanz, und der Anzahl der Wicklungen (= Zahl der Lagen). Zur Abgaberegulierung kann das Abgabesystem auch aus mehreren Varianten wie oben beschrieben bestehen. (z.B.: innen dick beschichtete PP-Folie, außen dünn beschichtetes Vlies).

Die Freisetzung der freizusetzenden Substanz erfolgt dadurch, dass diese von der Oberfläche und den Kanten der in aufgewickelter Form vorliegenden Polymermatrix in die Umgebung abgegeben wird. Als "Oberfläche" kommt hier natürlich nur der Abschnitt der Polymermatrix in Frage, der die "letzte Wicklung" darstellt und Kontakt zur Umgebung besitzt. Der unter dieser letzten Wicklung liegende Abschnitt der in aufgewickelter Form vorliegenden Polymermatrix besitzt keinen Kontakt zur Umgebung. Vielmehr ist dieser Abschnitt jeweils von einer nachfolgenden Wicklung abgedeckt. Die freizusetzende Substanz muss also bei ihrem Weg aus einem speziellen Abschnitt der Polymermatrix in die Umgebung zunächst in den Abschnitt der Polymermatrix eindringen, der jeweils die nächste Wicklung darstellt.

Eine Alternative besteht in der so genannten "Kantenfreisetzung". Hier wandert die freizusetzende Substanz innerhalb der Polymermatrix in eine Volumeneinheit am Rand der in aufgewickelter Form vorliegenden Polymermatrix, von wo aus sie in die Umgebung abgegeben wird. Diese Art der Freisetzung wird erzwungen, wenn ein Träger verwendet wird, der für die freizusetzende Substanz undurchlässig ist.

Die Herstellung des Abgabesystems erfolgt im Allgemeinen so, dass die Materialien, die die Polymermatrix bilden und die freizusetzende Substanz gemischt werden, ggf. mit weiteren Hilfsstoffen und ggf. unter Verwendung geeigneter Lösungsmittel und / oder Wärmeanwendung. Die dabei entstandene Masse wird in fließfähiger Form auf eine Unterlage aufgebracht und zu einem bahnförmigen Zwischenprodukt ausgestrichen. Das Unterlage kann ein bahnförmiges Material sein, das zu einem späteren Zeitpunkt verworfen wird oder vorzugsweise die im Abgabesystem als Träger fungierende Komponente. Nach dem Aufbringen der Masse wird diese ggf. getrocknet und / oder von gegebenenfalls vorhandenem Lösungsmittel befreit. Dabei entsteht die bahnförmig vorliegende Polymermatrix auf der Unterlage/dem Träger. Die Unterlage kann nun ggf. von der bahnförmig vorliegenden Polymermatrix abgezogen werden. In jedem Fall wird die bahnförmige Polymermatrix (mit oder ohne Träger) in eine Aufwickelvorrichtung eingeführt. Das Aufwickeln erfolgt vorzugsweise durch Auflegen eines zylindrischen Körpers auf die bahnförmige Polymermatrix, senkrecht zu deren Längsrichtung. Durch Drehen des zylindrischen Körpers und Transport der bahnförmigen Polymermatrix erfolgt das eigentliche Aufwickeln. Nachdem die gewünschte Zahl der Umdrehungen aufgeführt wurde, erfolgt durch Querschneiden ein Vereinzeln. Die nun in aufgewickelter Form vorliegende Polymermatrix wird anschließend in eine geeignete Verpackung transportiert und darin verschlossen.

Die folgenden Beispiele dienen der Erläuterung der Erfindung.

### Beispiel 1: Abgabesystem in Form eines Pheromondispensers

Ein Abgabesystem, welches im Weinbau als Pheromondispenser zur Verwirrung von Traubenwickler verwendet werden kann, wird nach dem allgemeinen Verfahren hergestellt. Als Träger wird ein einseitig alubedampftes PP-Vlies mit einer Breite von 40 mm und einer Länge von 3 m eingesetzt. Die Polymermatrix enthält 95 Gew.-% Durotak H 312 (ein handelsüblicher Hotmeltkleber) und 5 Gew.-% des Pheromons (E,Z)-7,9-Dodecadienylacetat. Die Beschichtungsbreite beträgt ebenfalls 40 mm bei einem Auftragsgewicht von 200 g/m². Die Polymermatrix ist um eine Aufhängevorrichtung (Haken) herum aufgewickelt.

Dieses Abgabesystem enthält 1,2 g des abzugebenden Pheromons.

### Beispiel 2: Abgabesystem in Form eines Duftstoffdispensers

Ein Abgabesystem wird als Duftdispenser zur Raumbeduftung hergestellt. Als Träger dient ein Vlies aus Viskose mit einer Dicke von 300 g/m², einer Breite von 100 mm und einer Länge von 20 m. Die Polymermatrix besteht aus 75 Gew.-% einer EVA-Copolymer- Kleberformulierung (Evathane 40-55) und 25 Gew.-% Citrusöl. Die Beschichtungsbreite beträgt 120 mm und das Auftragsgewicht der Polymermatrix 200 g/m². Der Verbund von Polymermatrix und Träger wird um eine Papphülse herum aufgewickelt.

Dieses Abgabesystem enthält 10 g abzugebendes Citrusöl.

### Beispiel 3: Abgabesystem in Form eines Lockstoffdispensers

Ein Abgabesystem wird als Lockstoffdispenser für die Anwendung in Insektenfallen hergestellt. Als Träger dient eine PE-Folie (Dicke 30 g/m², Breite 10 mm, Länge ca. 2,5 m). Die Polymermatrix besteht aus 99 Gew.-% eines handelsüblichen Hotmeltklebers (Durotak 380-2954) und 1 Gew.-% "Wein FK" (ein Lockstoffgemisch der Fa. Symrise), die mit einer Beschichtungsbreite von 10 mm und einem Auftragsgewicht von 70 g/m² auf den Träger aufgebracht wird. Es wird um eine Kunststoffhülse herum aufgewickelt.

Dieses Abgabesystem enthält 0,175 g abzugebenden Lockstoff.

### Die Abbildungen dienen der Erläuterung der Erfindung. Es zeigen:

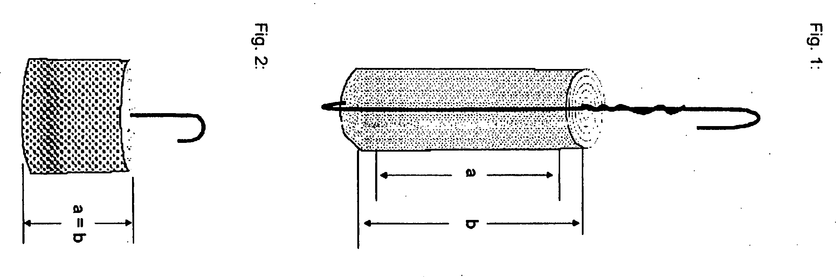

Fig. 1: Abgabesystem aus Polymermatrix mit Träger, bei dem der Träger eine Breite (b) aufweist, die größer als die Breite (a) der Polymermatrix ist.

Fig. 2: Abgabesystem aus Polymermatrix mit Träger, bei dem der Träger die gleiche Breite wie die Polymermatrix besitzt, d. h. (a) = (b).

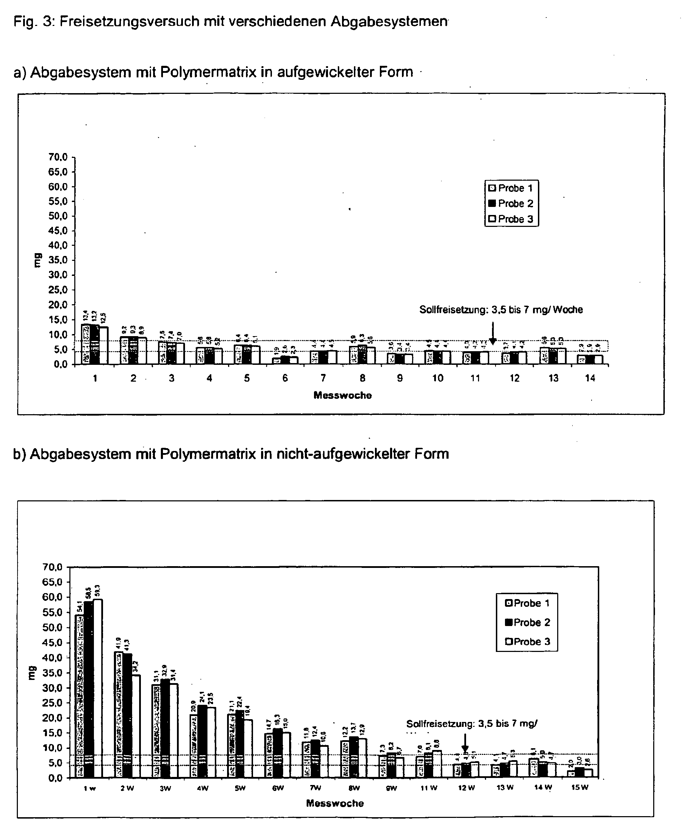

Fig. 3: Ergebnisse der Freisetzungsversuche. Das obere Diagramm a) zeigt die Freisetzung des Pheromons (E,Z)-7,9-Dodecadienylacetat im Verlauf von 14 Wochen aus Abgabesystemen mit einer in aufgewickelter Form vorliegenden Polymermatrix. Das untere Diagramm b) zeigt die Freisetzung des gleichen Pheromons aus einem entsprechenden Abgabesystem mit einer flachen, nicht-aufgewickelten Polymermatrix. Der Pfeil markiert den Bereich der "Sollfreisetzung" von 3,5 bis 7 mg/Woche. Man erkennt, dass dieser Bereich mit dem Abgabesystem mit der in aufgewickelter Form vorliegenden Polymermatrix (a) in der Zeit zwischen Woche 3 bis 13 erreicht wird. Das Abgabesystem mit der flachen, nicht-aufgewickelten Polymermatrix (b) erreicht diesen Bereich in der Zeit zwischen Woche 9 und 14. Dies bedeutet, dass die aufgewickelte Form der Polymermatrix über einen wesentlich längeren Zeitraum die Sollfreisetzung erreicht und insbesondere in den ersten Wochen nicht die übermäßig starke Pheromonfreisetzung der nicht-aufgewickelten Ausführungsform zeigt.

## Patentansprüche

1. Abgabesystem zur kontinuierlichen Freisetzung einer freizusetzenden Substanz, umfassend eine die freizusetzende Substanz enthaltende Polymermatrix, welche mindestens ein synthetisches Polymer enthält, und wobei es sich bei der freizusetzenden Substanz um eine flüchtige Substanz handelt, **dadurch gekennzeichnet, dass** die Polymermatrix in dem Abgabesystem in aufgewickelter Form vorliegt.

2. Abgabesystem nach Anspruch 1, **dadurch gekennzeichnet, dass** die Polymermatrix zusätzlich mindestens ein natürliches Polymer enthält.

3. Abgabesystem nach Anspruch 1 oder 2, **dadurch gekennzeichnet, dass** das synthetische Polymer thermoplastisch ist.

4. Abgabesystem nach einem oder mehreren der vorangegangenen Ansprüche, **dadurch gekennzeichnet, dass** die Polymermatrix eine Breite zwischen 2 mm und 50 cm besitzt, vorzugsweise zwischen 2 cm und 40 cm.

5. Abgabesystem nach einem oder mehreren der vorangegangenen Ansprüche, **dadurch gekennzeichnet, dass** die Polymermatrix eine Länge unterhalb von 200 m besitzt, vorzugsweise zwischen 5 cm und 20 m und besonders bevorzugt zwischen 20 cm und 5 m.

6. Abgabesystem nach einem oder mehreren der vorangegangenen Ansprüche, **dadurch gekennzeichnet, dass** die freizusetzende, flüchtige Substanz mindestens einen Lockstoff, ein Repellent, einen Duftstoff oder eine Kombination davon enthält.

7. Abgabesystem nach einem oder mehreren der vorangegangenen Ansprüche, **dadurch gekennzeichnet, dass** der Gehalt der freizusetzenden Substanz in der Polymermatrix zwischen 0,001 und 60 Gew.-%, vorzugsweise zwischen 0,1 und 35 Gew.% liegt.

8. Abgabesystem nach einem oder mehreren der vorangegangenen Ansprüche, **dadurch gekennzeichnet, dass** die in aufgewickelter Form vorliegende Polymermatrix zwischen 1 und 2000, vorzugsweise zwischen 10 und 500 Wicklungen aufweist.

9. Abgabesystem nach einem oder mehreren der vorangegangenen Ansprüche, **dadurch gekennzeichnet, dass** das System zusätzlich einen in flächiger Form vorliegenden Träger aufweist, der in aufgewickelter Form vorliegt.

10. Abgabesystem nach Anspruch 9, **dadurch gekennzeichnet, dass** der Träger die Polymermatrix einseitig vollflächig bedeckt.

11. Abgabesystem nach Anspruch 9 oder 10, **dadurch gekennzeichnet, dass** der Träger eine größere Breite (b) besitzt als die Breite (a) der Polymermatrix.

12. Verfahren zur Herstellung eines Abgabesystems enthaltend eine in aufgewickelter Form vorliegende, eine freizusetzende Flüchtige Substanz enthaltende Polymermatrix, umfassend die Schritte:
- Erzeugen einer Mischung aus einem synthetischen Polymer und mindestens einer freizusetzenden Substanz,
- Auftragen der Mischung auf eine bahnförmige Unterlage,
- Umwandlung der Mischung in eine die freizusetzende Substanz enthaltende Polymermatrix
- Aufwickeln der Polymermatrix
- Vereinzeln

13. Verfahren nach Anspruch 12, **dadurch gekennzeichnet, dass** die bahnförmige Unterlage vor dem Aufwickeln der Polymermatrix von dieser abgezogen wird.

14. Verfahren nach Anspruch 12, **dadurch gekennzeichnet, dass** die bahnförmige Unterlage der in flächiger Form vorliegende Träger des Abgabesystems ist.

15. Verfahren nach einem oder mehreren der Ansprüche 12 bis 14, **dadurch gekennzeichnet, dass** nach dem Aufwickeln ein in flächiger Form vorliegender Träger um die in aufgewickelter Form vorliegende Polymermatrix gerollt wird.

16. Verwendung eines Abgabesystems enthaltend eine in aufgewickelter Form vorliegende, eine freizusetzende flüchtige Substanz enthaltende Polymermatrix, welche mindestens ein synthetisches Polymer enthält als Duftstoffdispenser, als Pheromondispenser oder als Lockstoffdispenser.

## Claims

1. Delivery system for continuous release of a substance to be released, comprising a polymer matrix which comprises the substance to be released and which comprises at least one synthetic polymer, and the substance to be released being a volatile substance, **characterized in that** the polymer matrix in the delivery system is in wound form.

2. Delivery system according to Claim 1, **characterized in that** the polymer matrix further comprises at least one natural polymer.

3. Delivery system according to Claim 1 or 2, **characterized in that** the synthetic polymer is thermoplastic.

4. Delivery system according to one or more of the preceding claims, **characterized in that** the polymer matrix possesses a width of between 2 mm and 50 cm, preferably between 2 cm and 40 cm.

5. Delivery system according to one or more of the preceding claims, **characterized in that** the polymer matrix possesses a length of less than 200 m, preferably between 5 cm and 20 m, and more preferably between 20 cm and 5 m.

6. Delivery system according to one or more of the preceding claims, **characterized in that** the volatile substance to be released comprises at least one attractant, repellent, fragrance or a combination thereof.

7. Delivery system according to one or more of the preceding claims, **characterized in that** the amount of the substance to be released in the polymer matrix is between 0.001% and 60% by weight, preferably between 0.1% and 35% by weight.

8. Delivery system according to one or more of the preceding claims, **characterized in that** the polymer matrix in wound form has between 1 and 2000, preferably between 10 and 500 winds.

9. Delivery system according to one or more of the preceding claims, **characterized in that** the system further comprises a flat support in wound form.

10. Delivery system according to Claim 9, **characterized in that** the support covers the polymer matrix on one side over the whole area.

11. Delivery system according to Claim 9 or 10, **characterized in that** the support possesses a width (b) greater than the width (a) of the polymer matrix.

12. Process for producing a delivery system comprising a polymer matrix which is in wound form and comprises a volatile substance to be released, comprising the steps of
- producing a mixture of a synthetic polymer and at least one substance to be released,
- applying the mixture to a sheetlike substrate,
- transforming the mixture into a polymer matrix comprising the substance to be released,
- winding the polymer matrix, and
- singularizing.

13. Process according to Claim 12, **characterized in that** the sheetlike substrate is removed from the polymer matrix before said matrix is wound.

14. Process according to Claim 12, **characterized in that** the sheetlike substrate is the flat support of the delivery system.

15. Process according to one or more of Claims 12 to 14, **characterized in that** after the winding operation a flat support is rolled around the polymer matrix which is in wound form.

16. Use of a delivery system comprising a polymer matrix which is in wound form, comprises a volatile substance to be released and comprises at least one synthetic polymer as a fragrance dispenser, as a pheromone dispenser or as an attractant dispenser.

## Revendications

1. Système de distribution pour la libération continue d'une substance à libérer, comprenant une matrice polymère contenant la substance à libérer, qui contient au moins un polymère synthétique et où il s'agit, pour la substance à libérer, d'une substance volatile, **caractérisé en ce que** la matrice polymère se trouve sous forme enroulée dans le système de distribution.

2. Système de distribution selon la revendication 1, **caractérisé en ce que** la matrice polymère contient en outre au moins un polymère naturel.

3. Système de distribution selon la revendication 1 ou 2, **caractérisé en ce que** le polymère synthétique est thermoplastique.

4. Système de distribution selon l'une ou plusieurs des revendications précédentes, **caractérisé en ce que** la matrice polymère présente une largeur entre 2 mm et 50 cm, de préférence entre 2 cm et 40 cm.

5. Système de distribution selon l'une ou plusieurs des revendications précédentes, **caractérisé en ce que** la matrice polymère présente une longueur inférieure à 200 m, de préférence entre 5 cm et 20 m et de manière particulièrement préférée entre 20 cm et 5 m.

6. Système de distribution selon l'une ou plusieurs des revendications précédentes, **caractérisé en ce que** la substance volatile à libérer contient au moins un appât, un répulsif, un parfum ou une combinaison de ceux-ci.

7. Système de distribution selon l'une ou plusieurs des revendications précédentes, **caractérisé en ce que** la teneur en substance à libérer dans la matrice polymère se situe entre 0,001 et 60% en poids, de préférence entre 0,1 et 35% en poids.

8. Système de distribution selon l'une ou plusieurs des revendications précédentes, **caractérisé en ce que** la matrice polymère se trouvant sous forme enroulée présente entre 1 et 2000, de préférence entre 10 et 500 spires.

9. Système de distribution selon l'une ou plusieurs des revendications précédentes, **caractérisé en ce que** le système présente en plus un support plat, qui se trouve sous une forme enroulée.

10. Système de distribution selon la revendication 9, **caractérisé en ce que** le support recouvre la matrice polymère sur une face, sur toute sa surface.

11. Système de distribution selon la revendication 9 ou 10, **caractérisé en ce que** le support présente une plus grande largeur (b) que la largeur (a) de la matrice polymère.

12. Procédé de fabrication d'un système de distribution contenant une matrice polymère se trouvant sous forme enroulée, contenant une substance volatile à libérer, comprenant les étapes :
- production d'un mélange d'un polymère synthétique et d'au moins une substance à libérer,
- application du mélange sur une sous-couche en forme de bande,
- transformation du mélange en une matrice polymère contenant la substance à libérer
- enroulement de la matrice polymère
- séparation.

13. Procédé selon la revendication 12, **caractérisé en ce que** la sous-couche en forme de bande est enlevée de la matrice polymère avant l'enroulement de celle-ci.

14. Procédé selon la revendication 12, **caractérisé en ce que** la sous-couche en forme de bande est le support plat du système de distribution.

15. Procédé selon l'une ou plusieurs des revendications 12 à 14, **caractérisé en ce qu'**après l'enroulement, un support plat est enroulé autour de la matrice polymère se trouvant sous forme enroulée.

16. Utilisation d'un système de distribution contenant une matrice polymère se trouvant sous forme enroulée, contenant une substance volatile à libérer, qui contient au moins un polymère synthétique comme distributeur de parfum, comme distributeur de phéromones ou comme distributeur d'appâts.
